# EUROPEAN PATENT APPLICATION

(11) **EP 0 610 991 A2**
(43) Date of publication of application: **17.08.1994**
(21) Application number: 94200220.5
(22) Date of filing: 03.02.1994
(51) Int. Cl.: A61B 17/00, A61B 17/22, A61B 17/36

(54) **Device for laser assisted transurethral resection of the prostate(TURP)**

(30) Priority: 08.02.1993 US 14814; 26.05.1993 US 67566; 07.07.1993 US 87981
(71) Applicant: Xintec Corporation, Oakland California 94607 (US)
(72) Inventor: Chou, Marilyn, Piedmont, California 94611 (US); Ngai, Kwok Hing, San Francisco, California 94116 (US); Rink, John Lewis, San Francisco, California 94133 (US); Tam, Herrick, San Francisco, California 94133 (US); Yu, King Jian Jun, Oakland, California 94601 (US)
(74) Representative: Hall, Robert Leonard

(57) **Abstract**

This invention is related to treatment of benign prostatic hyperplasia (BPH), and more specifically, to a device particularly suited for performing an improved laser assisted transurethral resection of the prostate (TURP). This invention is an angle firing fiber optic laser delivery device. The device has a firing tip (42) which has an insert (44) with a highly reflective surface (45) lying at a specific angle with respect to the central axis of the fiber (41). The laser energy is reflected out a central opening or window (49). Another embodiment features the firing tip of the invention mounted on a cannula member. Another embodiment of the invention features a fiber with a bias cut end surface with a reflective surface provided thereon such that radiant energy is internally reflected at an operative angle for performing the prostatectomy. Various combination reflection and internal reflectance embodiments are disclosed as well. Various embodiments allow for cooling of the fiber and firing tip in different ways, including the use of cooling vents (48) on the firing tip and the use of a hollow coaxial channel for supplying irrigation fluid to the firing tip of the invention. The device can also be positioned and manipulated precisely and conveniently with the use of the positioning device and the indexed rotatable coupling device used on various embodiments of this invention.

## Description

### FIELD OF THE INVENTION

This invention is related to treatment of benign prostatic hyperplasia (BPH), and more specifically, to a device particularly suited for performing an improved laser assisted transurethral resection of the prostate (TURP).

### BACKGROUND OF THE INVENTION

Benign prostatic hyperplasia or hypertrophy (BPH) refers to an abnormal increase in the size of the prostate gland, usually affecting men over the age of 50. Symptoms usually develop gradually as the enlarging prostate compresses around the urethra. Flow of urine is obstructed, and there is difficulty starting urination. Men with this condition often get out of bed four or five times every night to urinate, and even then they may not experience complete relief from the urge to void. The process of restriction to the point of becoming a problem can take from several months to several years. The bladder muscle which would normally be relaxed initially to let urine through the urethra is used to try to force urine through. That muscle can become distended and overdeveloped, causing abdominal swelling. Severe abdominal pain and inability to relieve oneself can result and would require immediate medical attention. It is estimated that over 450,000 transurethral prostatectomies are performed each year and that nearly one third of all men in the United States who reach the age of 80 require such treatment.

Medical technology has developed extensively in this area. Between the 17th and 19th centuries surgical procedures were developed for removal of gall stones as well as for the open prostatic enucleation to relieve bladder obstruction. By the early 20th century the use of the wire loop electrocautery instrument to perform transurethral resection of the prostate (TURP) provided an alternative to open suprapubic or perineal incision, as was often resorted to. Over the past 60 years, the modern-type resectoscope with viewing capabilities in conjunction with cutting or reaming with an electrocautery wire loop have become what has frequently been referred to as the "gold standard" for treating such disease. The process includes heating a wire loop by electrical resistance and gradually drawing the heated loop back and forth longitudinally within the prostate to cut and cauterize tissue, creating a series of furrows along the length of the prostate until the entire area of the urethra has been treated circumferentially. Cooling fluid is used to irrigate the area being treated to minimize the risk of heat-damage to internal organs or other tissue. This is typically sterile water or some type of glycine/sorbitol/mannitol solution. Saline solution, although otherwise biocompatible, may cause a short circuit in the device and is therefore not used in this procedure.

Such treatment is not without post-operative morbidity. While mortality rates have declined to less than one quarter of one percent, complications in a very consistently high percentage of men receiving electrocautery TURP therapy include the need for blood transfusions and absorption of irrigation solutions into the prostate which causes a host of complications including nausea, hypertension, seizures and even mild myocardial events. Other complications include mild to severe incontinence, erectile dysfunction and retrograde ejaculation. Additionally, the need for heavy doses of local or general anesthetic made that procedure even more complicated and less available to many patients.

Alternative treatments for treating BPH include drug therapy. A new product which shows some hope for success is finasteride. However, new drug approvals require years of testing and clinical research. Moreover, drug therapy is not appropriate for large numbers of men who have other medical problems and who have already been prescribed other types of drugs. It seems that at the present time, an efficacious and safe drug therapy is still several years away.

Only within the past few years has laser assisted TURP become possible. Recent developments in fiber optic laser surgical scalpels which fire to the side or at some specific angle, are easily manipulated by the surgeon through a lumen of an endoscope. This results in deep thermal necrosis of adematous tissue which can be removed by ablation, and cutting of prostatic venous sinuses is avoided, eliminating excessive bleeding and absorption of irrigating fluids. This procedure also requires the use of epidural or spinal anesthesia or, since the operations is of a fairly short duration, a combination of intraurethral local anesthetic as well as a mild or short-acting intravenous sedation.

Although the first useful lasers were developed in the 1960s, recent advances in laser and fiber optic delivery systems have greatly enhanced the use of this technology in the field of medicine. Today there are numerous types of laser systems designed for operation in a wide range of applications primarily related to surgical and other medical procedures. A common type of laser known as a CO2 laser delivers radiation with a wavelength of 10.64 microns. However, in order to focus or channel the radiated energy produced by a CO2 laser it is necessary to configure sets of mirrors in certain ways. These systems are typically large and expensive. With the advent of the Nd:YAG type laser delivering electromagnetic energy at a wavelength of 1.064 microns, it became possible to generate and focus the laser radiation through a silica core optical fiber. Thus, fiber optic surgical tools have become important in certain procedures. The range of their utility is still being explored and discovered.

Laser fibers are used in different ways, including incision, necrosis or killing of live tissue, excision or removal of tissue and structure, and cauterization of tissue. A very focused beam would provide the greatest amount of control during either operation. Cauterization and necrosis of living tissue is accomplished by coagulation, or more precisely with respect to the laser itself, by photocoagulation of contacted or penetrated tissue. In this process the laser beam causes the proteins in the contacted tissue to heat up rapidly and thermally denature. This essentially kills living tissue and seals blood vessels. The process has been likened to frying an egg. In practice, during an incision procedure cauterization of the incised tissue is likely to occur simultaneously. Thus, laser surgery is often characterized by an absence of bleeding during the surgery.

In the prior art there are described devices which generate a dual wavelength beam of radiation and effect both cutting and cauterizing simultaneously. Such devices generally use one type of laser with some type of harmonic generator for providing half or double fundamental wavelength beams. There also exist inventions which deliver energy at much shorter wavelengths, such as 250-350 nm. At these wavelengths proteins, as opposed to water molecules, absorb the radiation. These systems, however, are less suitable for general types of surgical operations since they are more complicated to operate. Use of such systems has not become standard in most medical facilities and their cost is generally too high to justify their purchase for occasional use in fairly specialized procedures.

The construction of optical fibers used in surgical procedures is fairly simple. A quartz, plastic or silicone cladding is used to constrain the laser light to the quartz core. Theoretically, only a few of the entering photons are directed straight down the axis of the fiber. Transmission of the radiant beam is possible since the rest of the photons are constrained to the core of the fiber due to internal reflectance by the quartz cladding interface. Very few photons escape the fiber. The technology related to the use of silica core fibers in medical lasers is well known, e.g. B.P. McCann, Photonics Spectra, May 1990, pp 127-136. Differences between these types of optical fibers and those used in telecommunications and data transmission are important. Several design factors must be considered such as sterilizability, quartz core integrity and purity, power capacity and index of refraction of materials of construction.

Generally, 10 to 100 watts of energy are used to perform soil tissue surgery. A fiber optic laser scalpel used externally might be operated much differently than one used in internal or endoscopic surgery. Some endoscopes have multiple channels to accommodate a viewing port or camera, a laser delivery device, and an irrigation supply and accompanying vacuum channel. Delivery of high power radiation can have a very damaging effect on the fiber tip itself. One of the problems with existing designs is that the tip which directs the laser beam to a right angle becomes overheated. This is caused by an absorption of power (heat) at the reflecting surface. Overheating at or near the surface of the fiber tip can be caused by an accumulation of incompletely burned tissue which rapidly heats up and triggers a process known as thermal runaway. As heat builds up, the fiber tip gets hot and sometimes starts to melt or deform. Often, angle firing fiber optic surgical devices will need to be replaced partway through the surgical operation due to this problem.

Thus, the problems associated with currently available angle delivery fiber optic laser devices are mainly related to fiber overheating and failure. One solution would be to provide a transparent, hard, heat resistant tip, such as sapphire or quartz. An alternative is to provide a highly reflective surface in the scalpel tip for deflecting the beam.

Multiple layer optical interference films, also known as interference filters or films, are well known in the art. Such films comprise alternating layers of two or more materials, typically one with a relatively high index of refraction and the other with a relatively low index of refraction. These materials are also known as dielectrics. Such are well known in the art and can be designed to reflect or transmit light radiation from various portions of the electromagnetic spectrum. Often, materials with high and low indices of refractivity are applied in alternating layers so as to comprise a "quarter wave stack", each layer having an optical thickness equal to approximately one quarter wavelength of the incident light wave. These types of reflectors have been described providing optical absorption losses of as little as 0.0001% to 0.0002%.

Methods for manufacturing these films are described in the prior art. U.S. Patent Number 4,925,259, incorporated herein by reference, describes a damage-resistant dielectric coating formed over a silica substrate. Using a pulsed-plasma assisted chemical vapor deposition process several hundreds and even thousands of layer pairs can be deposited rapidly. Larger differences between the indices of refraction require a lesser number of layer pairs to obtain a given value of reflectance. In some cases, the indices of refractivity of alternating materials can be very similar and the number of layers very great. These coatings seem to have superior damage-resistance to optical radiation, approaching the damage resistance of pure silica. For laser applications using high power, components can be made to withstand high energy flux densities. They are also resistant to abrasion. Since the materials are very similar in composition there are fewer problems associated with differences in thermal and mechanical properties. Peeling and scaling is avoided as are microcracks which, in a given layer, would otherwise occlude the film.

At the reflecting surface, if most of the incident radiation is reflected very little will be absorbed and the temperature at the surface will not rise significantly. There is no known prior art providing an efficient reflector in intimate contact with the polished bias cut fiber tip surface, a reflective coating such as an interference film to internally reflect the beam of a laser used in conjunction with an optical fiber to perform surgical or other cutting or heating procedures, or a fiber tip with a sealed air pocket such that the laser light is reflected to the side based on the different indices of refraction of the optical fiber waveguide and the air or other gas or fluid in the pocket.

The international patent application published by the European Patent Office as International Publication Number WO 93/12728 is related to what is now well known and commonly practiced today in the field of laser assisted TURP. Currently, the standard laser assisted TURP is performed using an Nd:YAG laser or other suitable energy source. This would typically require suitable anesthesia or sedation and insertion of a 21 French or other appropriately sized cystoscope. The length of the prostate as well as the enlarged portion thereof would first be measured. When performing the laser prostatectomy, the standard approach is to insert the tip of a right angle firing fiber into the prostate through the cystoscope and to deliver or fire energy at approximately 4 points. These "round the clock" positions, most suitable for standard lateral lobe hypertrophy, are typically at the 2,4,8 and 10 o'clock positions. Starting at any one of the 4 mentioned positions and moving eventually to each one, the continuous wave Nd:YAG laser would require approximately 60 seconds of firing at a power rate of 60 watts to coagulate the tissue up to a depth of about 1.3 centimeters from the inner surface. Additional "around the clock" series' of coagulating burns would be performed for every approximately 4.5 centimeters of length of the enlarged prostate. At this point, a 16-F or equivalent Foley type catheter would be inserted and the patient would remain in recovery or be sent home.

Several shortcomings of these prior methods for laser assisted TURP exist. Often small mucosal bleeders are created by rubbing the tip of the fiber against the mucosal surface, requiring spot applications of laser energy to cauterize the wounds. A catheter must be placed in the urethra to drain the bladder, possibly during the operation, to remain in situ for at approximately 24 to 48 hours and sometimes up to one week. Hospital stays are between 2 and 5 days for most men. Immediately after the operation and for up to several days afterwards patients develop an acute phase of local prostatic edema or swelling, requiring the use of the above mentioned catheter. Additionally, not until removal of the catheter can a patient void naturally, and then, sometimes not until up to 6 months post-operation, are peak flow rates achieved. This is due to the long period post operation when the coagulated prostatic tissue dissolves and sloughs off. Insertion and wearing of the catheter is always accompanied by a risk of trauma to the urethra in the form of tears, cracks or irritation. For this reason, bacterial infection is always a concern and often requires the use of antibiotics to prevent complications.

### SUMMARY OF THE INVENTION

One embodiment of this invention features an insert in the firing tip of the present device. The insert is made out of gold or some other material which can be polished very highly. The insert could also have a coating or layer of reflective material applied to it. The insert can be polished before it is inserted into the firing tip. Thus, the precise angle at which the laser beam is directed, whether it be greater, equal to or less than 90 degrees to the incident beam, can be specified by the manufacturer. Also, the optical surface of the insert can be given a curvature for focusing the beam. The region in which a laser beam is focused will be the hottest, but other points in the beam path are useful for deep tissue coagulation or surface cauterization. Thus, firing tips can be manufactured with inserts with concave reflecting surfaces with varying focal lengths which will provide a greater range of precision instruments The surgeon can select the tool depending on the focal point desired and use the same instrument to incise, remove tissue or cauterize.

Another embodiment disclosed is a device wherein the end of the optical fiber is bias cut and, optionally, polished, and placed in intimate contact with a highly reflective mirrored surface. Depending on the application and operational parameters the instrument is designed around, it may be advantageous to bury the bias cut tip of the optical fiber into the reflective mirrored surface of the reflective cap or insert. Thus, the supplied laser radiation is reflected to the side and leakage of light near the interface between the fiber and the reflective surface is reduced or eliminated. Another embodiment of this invention provides the firing tip with a void or pocket of air at the end of a bias cut fiber. In this embodiment, the end surface of the firing tip might be coated with an interference film completely opaque to light at the wavelength of the laser beam.

An embodiment which has proved to be very effective is a truncated ball tip fiber having a bias cut through the ball portion providing a cut surface with a greater surface area than that of the fiber alone. When the tip is cut at an operative angle and polished, a laser beam is reflected internally to the side. The polished end surface can be placed in intimate contact with an efficient reflector such as a mirrored surface having a layer of gold or silver or other metal or material. The result would be to reflect any part of the laser beam which passed through the cut end surface and was not internally reflected. Additionally, the cut surface of the ball tip can be recessed or buried slightly in the reflective surface resulting in a device which transmits the laser beam in a defined angle without overheating or failing.

Another embodiment might have a reflective layer deposited directly onto the cut surface of the fiber. One material capable of being deposited in a very thin coating and producing a very high reflectance is gold. A protective layer over the reflective material could also be applied and be useful to add durability and thermal resistance to the reflective material. U.S. Patent Number 4,992,087, incorporated herein by reference, discloses a reflective coating consisting of a metal or metal alloy and a process for applying it to a glass surface.

The first step in a standard laser assisted TURP is to coagulate portions of the enlarged prostate. A laser assisted TURP is best performed with a pulsed wave laser delivery system, although almost any continuous wave (CW) laser could also be used. At a cycle rate of 60 Hz and with a peak power of approximately 1 kilowatt per pulse, the pulsed laser delivering an average of 45 watts can coagulate tissue approximately as efficiently as a CW laser delivering 60 watts.

Once the adematous prostatic tissue in each zone around the urethra through the length of the enlarged portion has been coagulated, a second pass using higher energy will vaporize the subject tissue. Using a pulsed laser, this vaporization can be accomplished at an average power rate of 60 watts. A CW laser would typically require 80 to 90 watts to effect vaporization of the tissue.

During this second pass of the fiber, either some or all of the previously coagulated tissue can be removed. It is possible to trench a single deep area along the length of the prostate at the 6 o'clock position, and often, this is all that is necessary to provide a patient with the ability to void sufficiently. Multiple channeling may be necessary in other patients, affected by a "painting" action with the tip of the fiber by the surgeon. Additionally or optionally, the coagulated tissue can be removed with a standard surgical resectoscope using mechanical cutting or reaming. After a few hours in the recovery room, if not immediately, the catheter can be removed. The inevitable swelling is generally not great enough to create a problem with voiding, even immediately after the operation. Additionally, as the typical TURP patient is between 50 and 80 years old, complications due to poor health or interactions with certain cardio-related medications, etc. are not infrequent. This operation can be performed on a virtually outpatient basis, thus eliminating any significant post-operation hospitalization.

Recovery from standard laser assisted TURP treatment lasts up to 6 months during which time a steady progressive enzymatic tissue liquefaction of the central coagulonecrotic tissue occurs and urine discharge flow rates return to normal. In fact, the average patient is not improved from their pre-operation symptoms. They may actually become worse. With this improved method, this entire healing period is reduced to a maximum of 7 weeks and patients experience complete relief almost immediately. In some studies, no episodes of bleeding have been reported during the healing period as opposed to the common observation, following laser assisted TURP, of an initial or terminal drop of blood with urination.

In the past, at approximately 18 hours post operation, serum prostate specific antigen (PSA) levels have been detected to peak, followed by a very gradual decline over the next few months back to baseline levels or lower. This fluctuation in serum PSA has been observed to parallel the pathophysiology of the thermal injury produced by the laser - an initial acute event with capillary leak causes tissue edema and is followed by gradual destruction of the prostate parenchyma. With this improved method, since tissue is destroyed, the pathology of remaining tissue can be determined with a needle biopsy. PSA levels, while still peaking somewhat after the operation, return to normal post prostatectomy levels more rapidly. Thus, unsuspected early stage malignancies can be detected earlier by observing a slow return to normal, or other variations, in the levels of serum PSA. Finally, patients on blood thinners can resume their medication fairly promptly as opposed to having to wait for up to one week, as previously necessary.

A scalpel which could be adjusted to provide the precise amount of cooling ability would be very useful. For example, if during a single operation, the surgeon first wished to coagulate a large amount of tissue, an instrument which delivered lower power radiation for relatively long periods of time would need to be well irrigated to prevent overheating from extended use. Thus large cooling vents could be necessary in the firing tip of such a scalpel. Then, to vaporize the tissue the surgeon could increase the power output of the scalpel and vaporize the coagulated tissue in short duration, high powered operation. Unless the flowrate of cooling water could be reduced, the vaporization step would be impossible. As described above, the two different processes of coagulating and ablation require different surgical operating parameters.

Another problem associated with current laser scalpels is that they are often clumsy to use and difficult to manipulate precisely. One problem is that the quartz fiber is so thin it is difficult to grasp effectively, especially if it is used in conjunction with a cystoscope or some type of endoscope where the firing end cannot be controlled directly by the surgeon. Also, as the scalpel is rotated and manipulated by the surgeon, the fiber becomes twisted under a certain amount of angular torque. It would be desirable to provide a scalpel which would be easily controlled, perhaps through the use of some external control means.

It is an object of this invention to provide a device suitable for performing a laser assisted transurethral resection of the prostate.

It is an object of this invention to provide a device for performing laser assisted transurethral resection of the prostate whereby the patient is subjected to a minimal amount of pain and discomfort.

It is an object of this invention to provide a device for performing laser assisted transurethral resection of the prostate which is performed in a clinic or in a hospital on virtually an out patient basis with no significant trauma or other post-operation morbidity requiring hospitalization.

It is an object of this invention to provide a procedure for performing laser assisted transurethral resection of the prostate whereby no catheter is required to be left in the urethra after the operation for any significant period of time, thereby permitting normal voiding of the bladder immediately following the operation, less chance of infection with an attendant need for antibiotics or other drug therapy.

It is an object of this invention to provide a device for performing laser assisted transurethral resection of the prostate whereby the excess tissue of the enlarged prostate is first coagulated and then vaporized or ablated, eliminating the lengthy period of tissue dissolving and sloughing off normally associated with such procedures.

Clinical applications for this invention include surgical ablation, vaporization, incision, excision, coagulation and cauterization of tissue. These operations can be performed in air or in fluid, either in open or in endoscopic methods, through natural body channels or through artificial incisions. Other applications include scientific industrial, entertainment, communications, and other commercial applications where angle delivery of laser beams via optical fibers at any wavelength is useful.

Numerous other advantages and features of the present invention will become readily apparent from the following detailed description of the invention and the embodiments thereof, from the claims and from the accompanying drawings in which the details of the invention are fully and completely disclosed as a part of this specification.

### BRIEF DESCRIPTION OF THE DRAWINGS

Figure 1 is a schematic view of a resectoscope inserted through the urethra and positioned adjacent to the prostate gland.

Figures 2 and 3 are schematic views of two embodiments of the invention.

Figures 4 and 5 are schematic views of two embodiments of the invention with a reflective surface on an insert in the firing tip.

Figures 6, 7 and 8 are schematic views of three embodiments of the internal reflectance devices of this invention.

Figures 9, 10, 11 and 12 are schematic views of four embodiments of the invention with a reflective surface on an insert in the firing tip.

Figures 13, 14, 15 and 16 are schematic views of four embodiments of the firing tip of this invention.

Figure 17 shows how the reflected energy can be directed to an angle of approximately, somewhat greater than and somewhat less than 90 degrees with respect to the central axis of the fiber, respectively.

Figure 18 shows the reflected beam pattern of the radiant energy impinging upon a flat, planar surface and upon a concave curved surface, respectively.

Figure 19 shows the positioning device.

Figure 20 shows the indexed, rotatable coupling device attached to a cannula mounted firing tip.

### DETAILED DESCRIPTION OF THE INVENTION

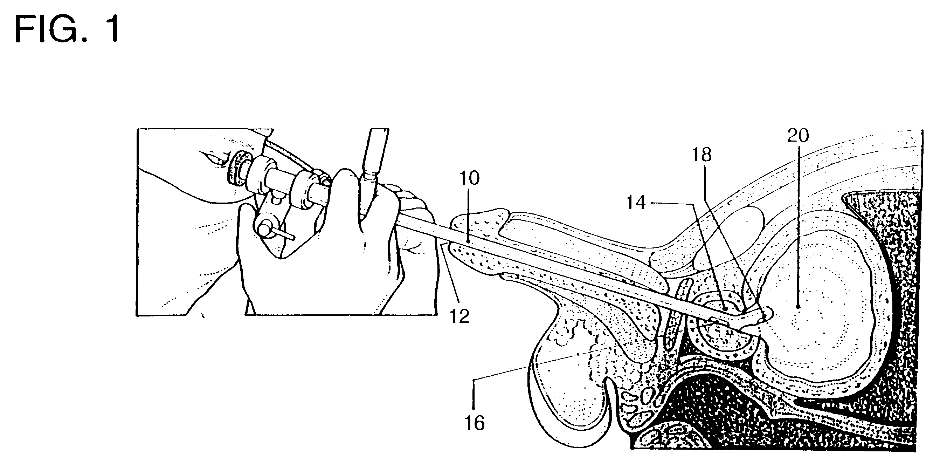

Figure 1 is a schematic view of a resectoscope **10** inserted through the urethra **12** and positioned adjacent to the prostate gland **14**. Any suitable cystoscope or resectoscope can be used and various types are readily available. An opening at the end **16** allows for the laser energy to be directed directly onto the tissue of the enlarged prostate from a fiber optic laser delivery device inserted through the scope. Another opening **18** might be used for viewing or for irrigation and cooling of the general area being lased. The bladder **20** is indicated. Viewing capability is very important for precise operation and manipulation of the equipment. The surgeon will generally measure the prostate gland in order to determine its length, an important factor in the operation. Figure 1 shows one type of resectoscope through which the surgeon has direct view of the operation. Some modern resectoscopes also allow the operation to be viewed on a large screen transmitted by a camera with a lens in the tip of the resectoscope. There are many different types of cystoscopes and resectoscopes currently on the market.

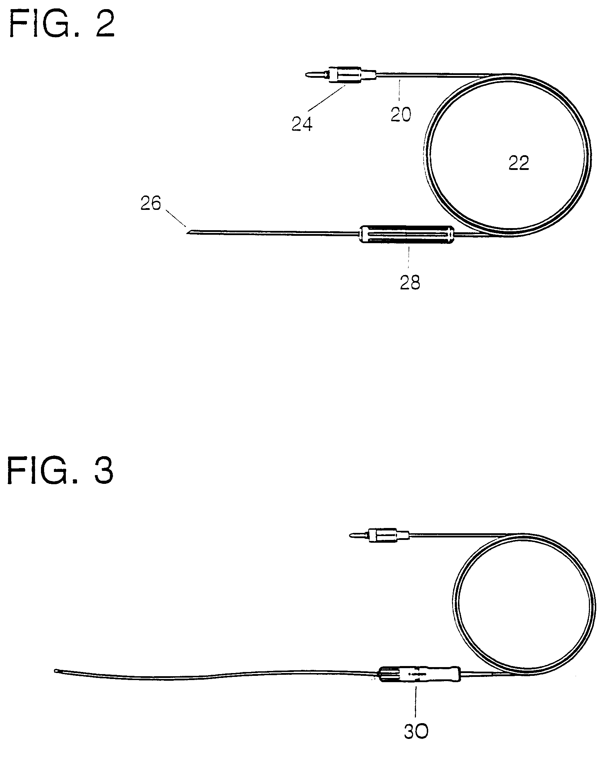

Figure 2 is a view of the present invention, a surgical scalpel. At the proximal end **20** of the optical fiber **22** there is a releasable optical fiber connector **24**. These connectors are standard in the industry and can also be proprietary. At the distal end of the assembly there is the firing tip **26**. Also shown is a positioning device **28** for use when the scalpel is operated with a lumen or endoscope or in another type of procedure. The distance through which the scalpel is inserted into a rigid cannula or endoscope can be adjusted and precisely positioned by the surgeon during a surgical operation. The device can be any apparatus which can be precisely positioned on the flexible fiber and will be convenient to use without hampering the operation of the scalpel meanwhile aiding the surgeon. It can be thought of as a handle or clamping system for the fiber. One such device would be made of two sections which screw together. As the two parts screw together they would clamp or pinch onto the fiber itself. Thus, the positioning device would be attached to the fiber and would be convenient for manipulating the firing tip precisely. The device could also have a fine adjustment for metering a precise length of fiber through the positioning device. Figure 3 shows another type of such positioning device.

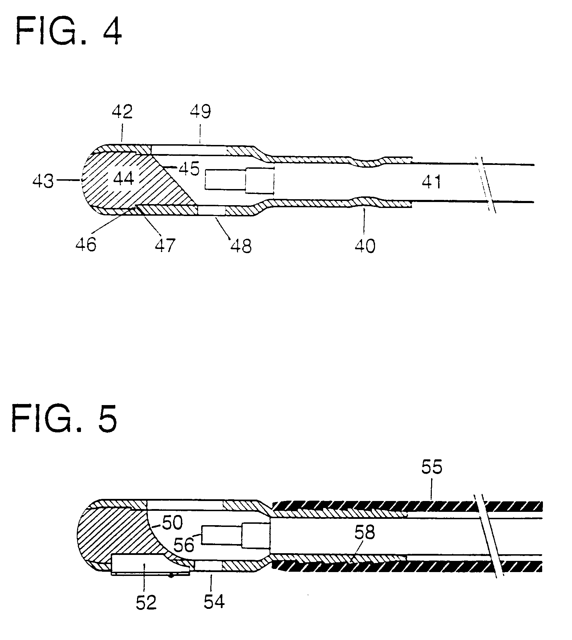

Figure 3 is a schematic view of an embodiment of the invention similar to that shown in Figure 2 except that the fiber is held in place within a cannula by a rotatable coupling device **30**. This cannula member is a semi-rigid tube. As the cannula member is turned about the central axis, the firing window changes orientation and the reflected laser energy is directed to a different axial position. The firing tip of this embodiment is shown in greater detail in Figure 5.

Figure 4 is a cross section view of a preferred embodiment of the firing tip. At the end of the firing tip on each side there is a means **40** for securing the firing tip to the optical fiber **41**. It will be understood by those skilled in the art that various means of attachment, such as a mechanical crimp (shown), molded construction, adhesives or clamps may be used. Figure 4 also shows the hollow body portion **42** of the firing tip. The hollow body portion of the tip has a distal end **43**. Assembly of the firing tip involves placing the reflective insert member **44** inside the hollow body portion. The distal end of the firing tip is formed so as to provide a smooth surface. This can be done by various means, such as mechanical compression, grinding, polishing or coating the tip. The tip can be rounded to provide ease in operation. The reflective surface **45** of the inserted member can be formed in various ways, including deposition, plating or sputtering with a reflective material (such as gold) upon the surface of the insert, mechanically compressing, grinding or polishing the insert, or other means known by those skilled in the art. The highly reflective surface of the insert permits very efficient transmission of the incident laser beam. Thus, very little of the incident radiation is absorbed by the insert member and overheating of the firing tip is prevented. The hollow body portion is constructed out of a durable, heat-withstanding material, such as stainless steel. Thus, as the insert is unavoidably heated to some extent by the incident radiant energy, the shape and integrity of the insert is maintained by the more durable firing tip body. Figure 4 also shows a machined boss **46** on the insert as well as a machined shoulder **47** on the inside surface of the central hollow body portion. When the boss is biased against the shoulder and the distal end of the hollow body portion is compressed around the insert member the insert member is held firmly in place. Also shown in Figure 4 is a cooling vent **48**. More than one cooling vent can be placed in this section of the hollow body portion. As the incident radiation is reflected out the firing window opening **49** of the firing tip the insert member becomes heated. As discussed above, a cooling solution is constantly used to irrigate the area being operated on. Thus, by a convective process, the cooling liquid is drawn through the cooling vents into the body portion. As the liquid is vaporized at the hot reflective surface of the insert, steam is generated normal to the reflective surface and escapes either through the cooling vent itself or through the firing window opening.

Figure 5 is a cross section view of another preferred embodiment of the firing tip. In this embodiment, the reflective surface **50** of the insert is somewhat concave, having a rounded or parabolic curvature. This feature allows the firing tip to be used either as a cutting or as a coagulating tool. The radiant energy beam impinges on the reflective surface, in a roughly elliptical pattern which is similar to that of the flat reflective surface embodiment. Then the beam is reflected into a cone shape, narrowing through a focal region, and thereafter widening. By increasing the radius of curvature of the concavity, the focal point of the incident beam can be extended to points farther away from the firing window of the firing tip. With this invention it is possible to provide the surgeon with a range of focal length tools. In the embodiment of Figure 5, the cooling vents have a movable shutter **52**. The shutter can be manually moved into a position covering part or all of the cooling vent **54**. This could also be a screen or cap, or other means known by those skilled in the art, to increase or restrict the cooling fluid flow rate through the cooling vents. Figure 5 also shows an embodiment of the invention with the firing tip secured to a cannula. This semi-rigid tube shaped cannula **55** covers the fiber which extends from the end of the cannula member and is positioned such that the transmitting end of the waveguide **56** is directly adjacent to the reflecting surface **50** of the firing tip. The attachment means **58** is shown here as a machined barbed end which engages the semi-rigid cannula member. It will be understood by those skilled in the art that alternative attachment means, such as adhesives or clamps, could also be used.

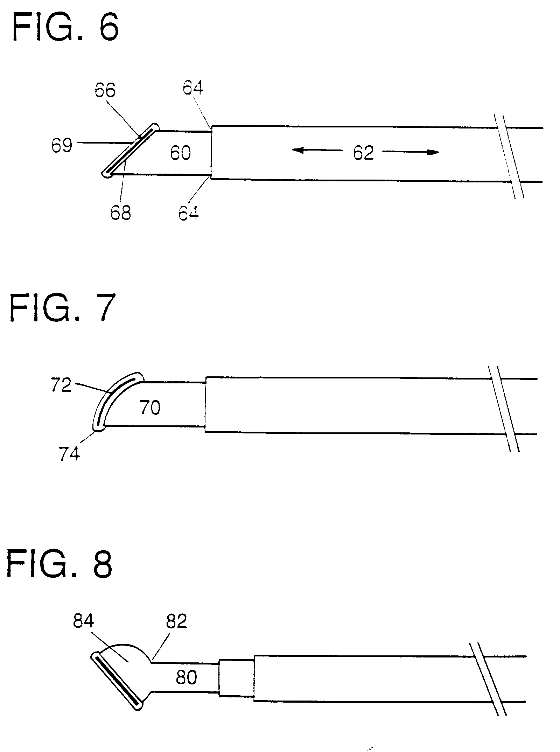

Figure 6 is a schematic view of another preferred embodiment of the firing tip of the scalpel. As shown, the fiber **60** is cleaved at an angle, other than perpendicular, to the central axis **62** of the fiber. The cladding of the fiber **64** is removed near the firing tip. The reflective coating **66** is only applied to the fiber end surface **68** exposed at the cleaved end of the fiber. The reflective layer may be some sort of interference coating comprised of several layers of materials with alternating high and low indexes of refraction. By varying the materials, thicknesses, and number of layers applied, very specific shape and directional patterns of reflectance can be produced by such coatings. With some materials, such as gold or other metals or materials, a minimum thickness is required. Also in Figure 6 is a protective layer **69** over the reflective coating. This layer would be durable and bond efficiently to the reflective layer. This coating can be applied by sputtering, vapor deposition, or in other ways known to those skilled in the art. For example, if the protective coating is a type of glass or ceramic the coating could be applied in a molten state or could be produced using some other high temperature process.

Figure 7 is a schematic view of another preferred embodiment of the firing tip. In this embodiment the cleaved end of the optical fiber has a curvature providing a rounded surface **70** to apply the reflective coating **72**. This curvature provides a reflective surface which will focus the beam through a narrow region. When the radiant energy beam impinges on the somewhat concave reflective surface the beam is reflected and forms an elliptical cone shaped beam, narrowing through a focal region outside of and beyond the end of the fiber, and thereafter widening. By increasing the radius of curvature of the reflecting surface, the focal point of the incident beam can be extended to points farther away from the firing window of the firing tip. Also shown is a protective layer **74** applied over the reflective coating. With this invention it is possible to provide the surgeon with a range of focal length tools which can coagulate as well as ablate tissue.

Figure 8 is a schematic view of another preferred embodiment of the firing tip. In this embodiment the diameter of the optical fiber waveguide **80** increases at a point **82** near the firing tip. One way to make this tip would be to heat the end of a 600 or 1000 micron diameter fiber so as to melt the quartz fiber itself. As the tip melts the molten silica will coalesce at the end and form a bead or drop of molten silica. Alternatively, the tip of a fiber could be fused to the end of a section of silica rod having a greater diameter than that of the fiber itself. Thus, laser energy will be transmitted through the fiber into the enlarged section **84**. This section can then be cleaved in a fashion similar to the embodiment of Figure 6. The optical interference coating and, if desired, the protective layer, can then be applied. The resulting tip will have an ellipsoidal shaped end, the face of which is larger than that produced by a cleaved fiber alone. Therefore, the laser beam produced upon reflection will have a greater diameter also. This embodiment could be useful when a relatively broader energy beam is required but a non-diverging beam is desirable. It will be noted that the enlarged-diameter section need not be longer than the equivalent of one or two diameters but that it could be considerably longer. Additionally, the end could be formed like that in Figure 7 so as to provide a diverging beam.

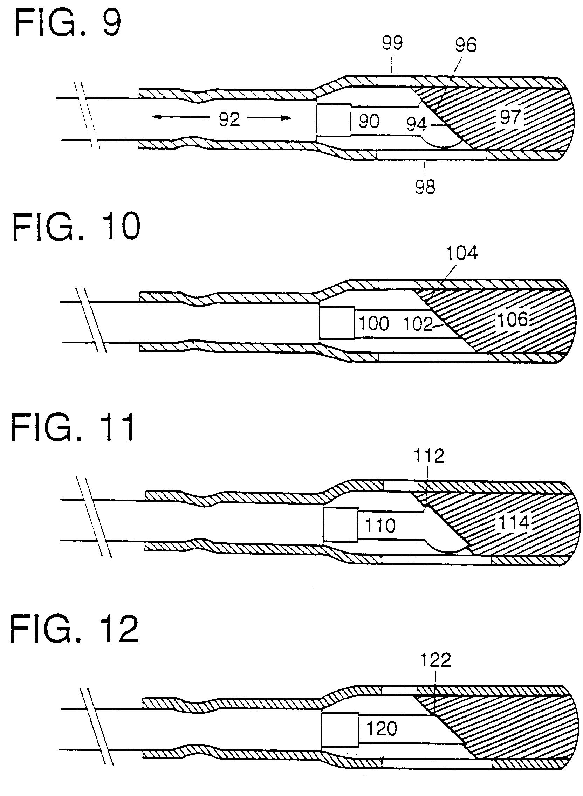

Figure 9 is a schematic view of another preferred embodiment of the fiber tip. As shown, the fiber with a ball end **90** is bias cut at an angle, other than perpendicular, to the central axis **92** of the fiber. The cladding of the fiber is removed near the distal fiber tip. The bias end surface of the fiber **94** is in intimate contact with the mirrored reflective surface **96** of insert **97**. The main laser beam delivery window **98** is oriented to an operative position with respect to the fiber and mirrored surface. A cooling vent **99** may or may not be provided. Thus, the mirrored reflective surface acts as both a heat sink and reflector during use. The cooling vent may or may not be used for irrigation fluid to be circulated around the fiber tip. The surfaces in contact with each other could be complementarily contoured so as to provide a beam with a specific pattern. For example, the polished tip of the fiber, while cut at an angle to the fiber's central axis, might also have a generally convex shape in intimate contact with a reflective cap with a corresponding concavity. This would result in a beam which focused at a point beyond the fiber and then thereafter became divergent. Contoured surfaces might be spherical, parabolic, ellipsoidal, etc.

Figure 10 is a schematic view of another preferred embodiment of the fiber tip. In this embodiment the optical fiber **100** does not have a ball tip. The end has been given a bias cut end surface **102**. The end is placed in intimate contact with the reflective surface **104** of insert **106**, as in Figure 9.

Figure 11 is a schematic view of another preferred embodiment of the fiber tip of the laser delivery device of this invention. This embodiment, similar to that shown in Figure 9, shows the bias cut ball tip fiber **110** buried slightly into a small recess **112** in the reflective surface. In this embodiment the reflective surface is on an insert **114** encased within the cap. This buried tip prevents leakage of light and makes transmission of laser energy more precise.

Figure 12 is a schematic view of another preferred embodiment of the fiber tip of the laser delivery device of this invention. This embodiment, similar to that shown in Figure 10, shows the bias cut fiber **120** buried slightly into a small recess **122** in the reflective surface. In this embodiment the reflective surface is on an insert encased within the cap. This buried tip prevents leakage of light and makes transmission of laser energy more efficient.

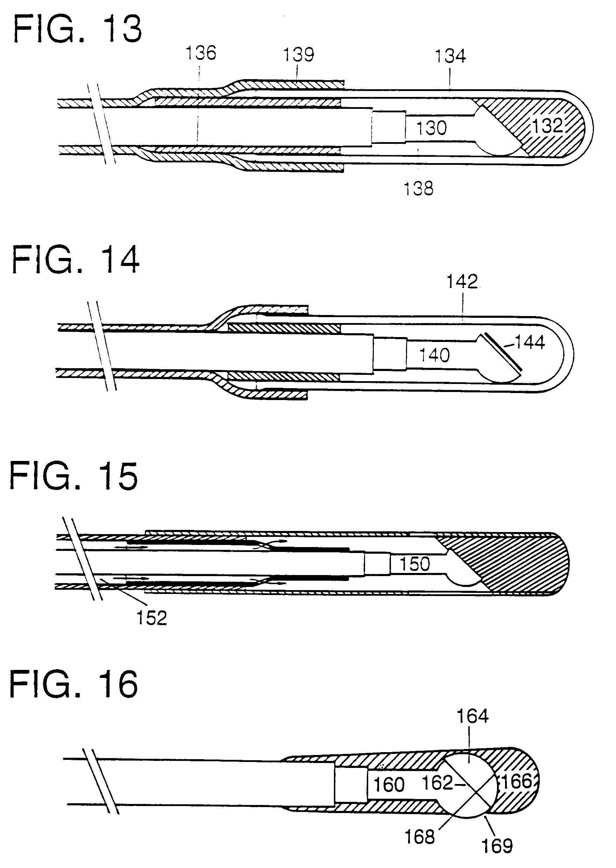

Figure 13 is a schematic view of another preferred embodiment of the fiber tip of the laser delivery device of this invention. In this embodiment a bias cut end surface of a ball tip fiber **130** is in intimate contact with a reflective mirrored insert **132** with a recess to prevent leakage of light and consequential overheating. The assembly is encased within a sealed transparent shell **134** with a bushing connection **136**. This shell may be made out of quartz, silica, pyrex or any suitable material transparent to laser light at any applicable wavelength and sufficiently heat resistant. The chamber **138** could be evacuated, filled with air or any other suitable fluid or gas which would result in beam reflection or refraction at predetermined angles. The bushing is constructed from a piece of material **139** clamped over the transparent shell to secure the assembly. This bushing connection provides increased mechanical integrity during operation, especially at elevated temperatures and when used with an irrigation or cooling system.

Figure 14 is a schematic view of another preferred embodiment of the fiber tip of the laser beam delivery device of this invention. In this embodiment a bias cut end surface of a ball tip fiber **140** is enclosed within a sealed transparent shell **142**. The bias cut fiber tip surface may also be coated with a reflective or interference film **144**.

Figure 15 is a schematic view of another preferred embodiment of the fiber tip of the laser delivery device of this invention. This embodiment, similar to that shown in Figures 5 and 11, shows the fiber **150** placed inside a coaxial cooling channel **152**. This embodiment is used when there is a source of cooling or irrigation fluid such as CO2 or saline. In operation, as the device is used the cooling or irrigation fluid is pumped through the coaxial section of the device and flow is shown by the arrows in FIG. 6. This maintains low temperatures near the fiber tip. This embodiment is used in conjunction with a laser source utilizing a fiber tip temperature detection and protection system.

Figure 16 is a cross section view of another preferred embodiment of the fiber tip of the laser delivery device of this invention. As in Figure 9, a ball-tip fiber **160** with a bias cut polished fiber surface **162** is obtained. The surface might optionally be coated with an interference film. A pocket of air or other gas or fluid **164** is provided by an end cap portion **166** of the fiber tip. The inside surface **168** of the end cap could be provided with a highly reflective or mirrored surface. Thus, while most of the laser beam will be reflected off the bias cut end surface of the fiber itself, any stray beam impinging upon the inside surface of end cap will also be reflected out the side window **169** of the device with the rest of the reflected beam.

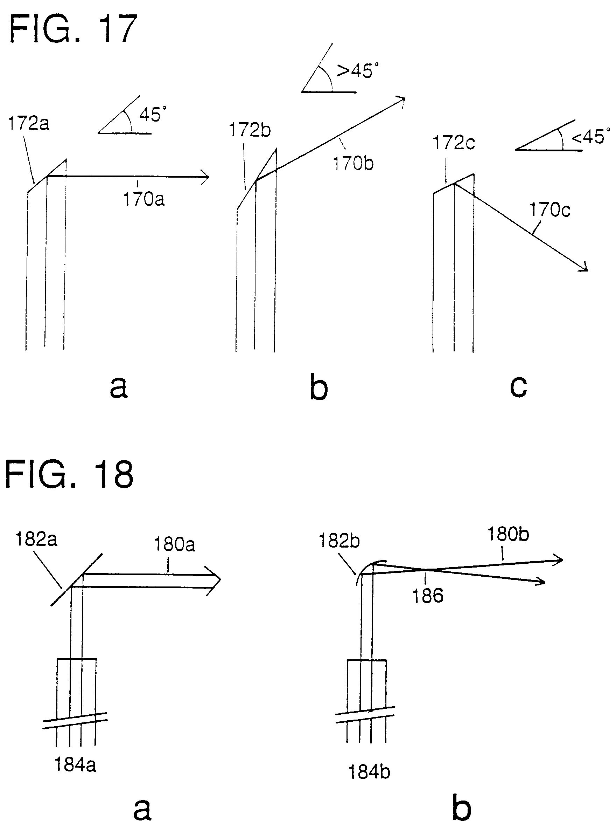

Figures 17a-c shows the reflected beam patterns of this invention. In Figure 17a, the laser beam path **170a** is directed to an angle of approximately 90 degrees with respect to the incident beam. The bias cut fiber **172a** has an angle of approximately 45 degrees with respect to perpendicular to the central axis of the fiber, as shown. In Figure 17b, the laser beam path **170b** is directed to an angle somewhat greater than 90 degrees with respect to the incident beam. The bias cut fiber **172b** has an angle of somewhat more than 45 degrees with respect to perpendicular to the central axis of the fiber, as shown. In Figure 17c , the laser beam path **170c** is directed to an angle somewhat less than 90 degrees with respect to the incident beam. The bias cut fiber **172c** has an angle of somewhat less than 45 degrees with respect to perpendicular to the central axis of the fiber, as shown.

Figures 18a-b shows the difference in reflected beam patterns. In Figure 18a, **180a** shows an embodiment of the current invention having a flat, planar reflective surface **182a**. In Figure 18b, **180b** shows an embodiment having a concave reflective surface **182b**. A cross sectional view is shown but it will be understood that the beam patterns emanating from the fibers near **184a** and **184b** are mostly cylindrical, being only slightly divergent when the tip is immersed in fluids. In the embodiments with a flat reflecting surface, the reflected beam is still only slightly divergent, maintaining a narrower shape and fairly uniform intensity throughout its length, in areas near the firing tip. However, embodiments with a concave reflecting surface will produce a reflected beam which is cone shaped, narrowing or converging through a region **186** corresponding to the focal point of the curved reflective surface, and thereafter widening. The cross section area of the beam pattern near **186** is very small and the beam is hottest near this focal point. Thus, cutting, tissue ablation or vaporization is possible when the scalpel of Figure 5 is positioned so that the laser beam impinges upon the tissue at a point near the focal point of the device. This embodiment may also be effective for coagulating tissue if the scalpel is positioned farther away from the tissue to be coagulated, such that the impinging beam is larger and less intense.

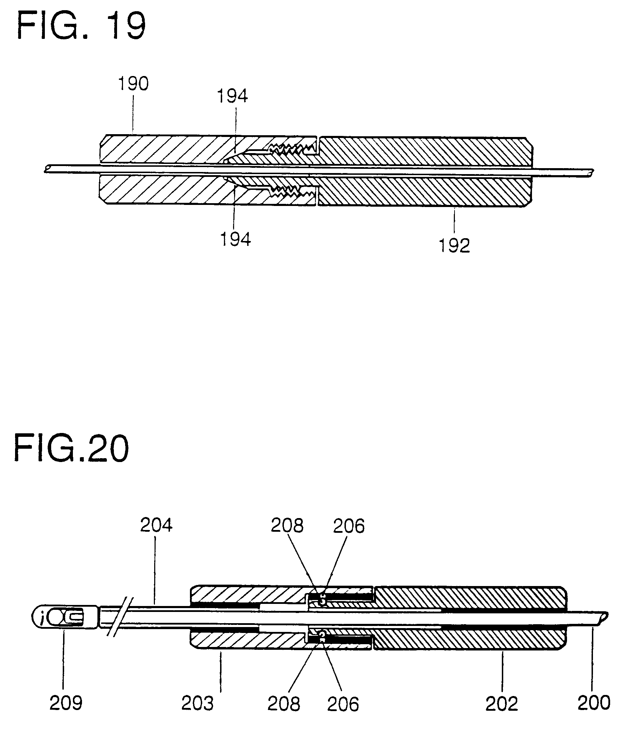

Figures 19 and 20 show positioning devices similar to those indicated by reference numerals **28** and **30** in Figures 2 and 3. With respect to Figure 19, the two parts of the device **190** and **192** screw together with the fiber passing directly through the center of the two parts. As part **190** threads onto part **192**, the tip of part **192** inside part **190** is compressed about the fiber at points **194**. As the compression is increased the device becomes locked onto the fiber. Thus, this positioning device allows the depth of insertion of the fiber into an external endoscope to be determined precisely. The fiber is inserted into the appropriate channel of the scope until the positioning device rests against the channel port and can be manipulated by the surgeon. The locking means described is one embodiment and other locking means would be known to those familiar with the art.

Figure 20 shows a fiber with firing tip and an indexed rotatable coupling with a cannula. The optical fiber **200** is shown passing through the two main parts of the device, an inner member **202** and a sleeve member **203**. The rigid cannula member **204** is attached to sleeve **203**. Inner member **202** locks onto the fiber while sleeve **203** rotates about the fiber. At points **206** there are small indentations or slots spaced evenly and circumferentially around the inside of sleeve **203**. Small stubs or key elements **208** located on the external surface of inner member **202** engage the slots of part **203**, thus locking the two parts **202** and **203** together. As sleeve **203**, coupled to the cannula member, is rotated about the fiber, the regularly spaced slots in part **203** are selectively engaged by the small stubs. Thus, a series of 6 regularly spaced slots in sleeve **203** will result in 6 indexed positions of rotation of the device. A different number of slots can be used to increase or decrease the degree of selectivity desired. As the cannula member is twisted into different positions, the firing window **209** (part of the firing tip of the cannula member) changes orientation and direction of fire. Only one possibility for the structure of the indexed rotatable coupling means is described here. Various embodiments of the indexed rotatable coupling means would be obvious to those skilled in the art.

## Claims

1. A fibre optic laser delivery device characterised in that it comprises means for deflecting the light to a predetermined angle to the axis of the waveguide, wherein the said means comprises a reflecting surface, said surface being positioned adjacent to the end of the waveguide, or in intimate contact therewith, and at an angle to the axis of the waveguide.

2. An angle firing fiber optic laser delivery device for cutting, coagulating and vaporizing tissue by means of radiant energy, characterised in that said device comprises:
a fiber optic waveguide having
a receiving end,
a central axis, and
a transmitting end;
and a firing tip, said firing tip comprising:
an attachment means for attaching said firing tip to said waveguide adjacent said transmitting end;
a central hollow body portion having a distal end and a proximal end,said attachment means attaching said central hollow body portion of said firing tip to said waveguide at said proximal end, said central hollow body portion partially cut away defining a central opening;
and a highly reflective surface, said highly reflective surface being positioned within said central hollow body portion of said firing tip in an operative position whereby said radiant energy is reflected through said central opening at an operative angle with respect to said central axis.

3. An angle firing fiber optic laser delivery device for cutting, coagulating and vaporizing tissue by means of radiant energy characterised in that, said device comprises:
a fiber optic waveguide having
a receiving end,
a central axis,
and a transmitting end;
a rigid cannula member, said cannula member having a proximal end and a distal end, said waveguide being axially disposed within said cannula member such that the transmitting end of said waveguide is adjacent to said distal end of said cannula member;
a waveguide locking means adjacent to said proximal end of said cannula member for locking said waveguide within said cannula member thereby maintaining said transmitting end of said waveguide adjacent to said distal end of said cannula member; and
a firing tip, said firing tip comprising:
an attachment means attaching said firing tip to the distal end of said cannula member;
a central hollow body portion having a proximal end and a distal end, said attachment means attaching said proximal end of said central hollow body portion of said firing tip to said distal end of said cannula member, said central hollow body portion partially cut away defining a central opening; and
a highly reflective surface, said highly reflective surface being positioned within said central hollow body portion of said firing tip in an operative position whereby said radiant energy is reflected through said central opening at an operative angle with respect to said central axis.

4. A combination reflectance angle firing fiber optic laser delivery device for cutting, coagulating and vaporizing tissue by means of radiant energy, characterised in that said device comprises:
a fiber optic waveguide having
a receiving end,
a central axis, and
a transmitting end, said transmitting end having a bias cut end surface, said end surface lying at an operative angle with said central axis of said fiber optic waveguide; and
a firing tip, said firing tip comprising:
an attachment means for attaching said firing tip to said waveguide adjacent said transmitting end;
a central hollow body portion having a distal end and a proximal end, said attachment means attaching said central hollow body portion of said firing tip to said waveguide at said proximal end, said central hollow body portion partially cut away defining a central opening; and
a highly reflective surface, said transmitting end in intimate contact with said highly reflective surface, said transmitting end and said highly reflective surface both being positioned within said central hollow body portion of said firing tip in an operative position whereby said radiant energy is reflected through said central opening at an operative angle with respect to said central axis.

5. The device of claim 4 characterised in that said reflective surface is provided with a recess such that said bias cut end surface of said fiber optic waveguide is disposed within said recess whereby any normal leakage of light is reduced.

6. The device of any of claims 1-5 characterised in that a highly reflective surface is placed on a first end of an insert member axially disposed within said central hollow body portion, a second end of said insert member being adjacent to the distal end of said central hollow body portion.

7. The device of claim 6 characterised in that said central hollow body portion further comprises an internal circumferential shoulder located at a point intermediate between said central opening and said distal end of said central hollow body portion, said insert member further has a circumferential boss at a point intermediate between said first end and said second end, said boss biased against said shoulder.

8. The device of any of claims 1-7 characterised in that said central hollow body portion of said firing tip further comprises a plurality of openings somewhat smaller in size than the central opening to act as cooling vents for said firing tip.

9. The device of claim 8 characterised in that it further comprises a flowrate restricting means movably attached to said central hollow body portion adjacent to said cooling vents.

10. The device of any of claims 1-9 characterised in that the reflective surface is a highly reflective planar surface or characterised in that the reflective surface is a highly reflective concave surface.

11. An internal reflectance angle firing fiber optic laser delivery device for cutting, coagulating and vaporizing tissue by means of radiant energy, said device comprising:
a fiber optic waveguide having:
a receiving end,
a central axis, and
a transmitting end;
and a firing tip, said firing tip comprising:
a bias cut end face on said transmitting end, said end face lying at an operative angle to said central axis of said waveguide; and a highly reflective surface applied to said end face whereby said radiant energy is reflected at an operative angle with respect to said central axis.

12. The device of any of claims 1-11 characterised in that said highly reflective surface consists of a dielectric material, and/or
characterised in that said highly reflective surface consists of a metallic material, and/or
characterised in that said highly reflective surface consists of a plurality of layers of material, said materials having different indices of refraction.

13. The device of any of claims 1-12 characterised in that the diameter of said fiber optic waveguide increases at a point near the firing tip of said device forming a truncated ball shaped firing tip.

14. The device of any of claims 1-10, 12 or 13 characterised in that said firing tip further comprises a protective layer applied over said central hollow body, said protective layer being composed of a material transparent to said radiant energy.

15. The device of any of claims 11-13 characterised in that said firing tip further comprises a protective layer applied over said highly reflective surface, said protective layer being composed of a material transparent to said radiant energy.

16. The device of claims 14 and 15 characterised in that there is a space defining a chamber interposed between said bias cut end surface of said fiber and said protective layer, said chamber being sealed by a sealing means.

17. The device of claim 16 characterised in that said chamber is filled with a fluid, or characterised in that said chamber is filled with a gas, or characterised in that said chamber is evacuated.

18. The device of any of claims 1-17 characterised in that said highly reflective surface lies in a plane at approximately 45 degrees with respect to said central axis such that radiant energy is reflected off of said highly reflective surface at an angle of 90 degrees with respect to said central axis, or characterised in that said highly reflective surface lies in a plane at more than 45 degrees with respect to said central axis such that radiant energy is reflected off of said highly reflective surface at an angle of more than 90 degrees with respect to said central axis, or characterised in that said highly reflective surface lies in a plane at less than 45 degrees with respect to said central axis such that radiant energy is reflected off of said highly reflective surface at an angle of less than 90 degrees with respect to said central axis.

19. The device of any of claims 1-18 characterised in that said receiving end of said waveguide further comprises a means for coupling said waveguide to a source of radiant energy.

20. The device of any of claims 1-19 characterised in that it further comprises a positioning device, said positioning device having a locking means for attaching said positioning device to said waveguide at a point intermediate said receiving end of said waveguide and said firing tip.

21. The device of claim 20, characterised in that the positioning device further comprises a rotatable section, said rotatable section being disposed between said locking means of said positioning device and said receiving end of said waveguide, said rotatable section being rotatable about said waveguide's central axis, and said rotatable section preferably being indexed so as to provide rotation into a plurality of predetermined positions.

22. The device of claims 1-21 characterised in that said fiber is disposed within a hollow coaxial channel delivering coolant fluid to the transmitting end of said fiber optic waveguide.
